(19) Europäisches Patentamt
European Patent Office
Office européen des brevets

(11) **EP 4 785 875 A1**

(12) **EUROPEAN PATENT APPLICATION**

(43) Date of publication:
**05.08.2026 Bulletin 2026/32**

(21) Application number: **26155755.7**

(22) Date of filing: **02.02.2026**

(51) International Patent Classification (IPC):
**A61B 8/08** (2006.01)   **A61B 8/12** (2006.01)
**A61B 8/00** (2006.01)   **A61B 34/20** (2016.01)

(52) Cooperative Patent Classification (CPC):
**A61B 8/0841; A61B 8/0883; A61B 8/12;**
**A61B 8/4209; A61B 8/5207; A61B 34/20;**
A61B 8/085; A61B 8/4488; A61B 2034/2051;
A61B 2034/301

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**GE KH LA MA MD TN**

(30) Priority: **04.02.2025 US 202563753524 P**
**17.11.2025 US 202519390988**

(71) Applicant: **Siemens Medical Solutions USA, Inc.**
**Malvern, PA 19355 (US)**

(72) Inventors:
• **KIM, Young-Ho**
**Princeton, NJ, 08540-6632 (US)**
• **HUH, Jaeyoung**
**Princeton, NJ, 08540-6632 (US)**
• **KAPOOR, Ankur**
**Princeton, NJ, 08540-6632 (US)**

(74) Representative: **HKW Intellectual Property PartG mbB**
**Theresienhöhe 12**
**80339 München (DE)**

(54) **AI-BASED GUIDANCE SYSTEM FOR INTRA-CARDIAC ECHOCARDIOGRAPHY MANIPULATION TO MAINTAIN CONTINUOUS THERAPY DEVICE TIP VISIBILITY**

(57) Systems and methods for estimating an incident angle and trajectory of therapy devices within Intra-cardiac Echocardiography (ICE) images in real time in order to provide guidance for ICE manipulation to maintain continuous therapy device tip visibility. An ICE imaging sequence is acquired. A pre-trained ultrasound foundation model is used to extract spatial and temporal features in the sequence of ICE images. A transformer based temporal deep learning model is used that integrates the extracted spatial while incorporating historical passing points and angular information to in order to track the catheter tip within the ICE imaging plane. The tip tracking transformer based temporal deep learning model improves upon existing incident angle and trajectory model estimations in ICE procedures by improving prediction accuracy and consistency.

Fig. 1

**Description**

STATEMENT REGARDING FEDERALLY SPONSORED RESEARCH OR DEVELOPMENT

This invention was made with government support under 1R01EB028278-01A1 awarded by NIH/NIBIB. The government has certain rights in the invention.

CROSS REFERENCE TO RELATED APPLICATIONS

This application claims the benefit of the filing date under 35 U.S.C. § 119(e) of U.S. Provisional Application Serial No. 63/753,524 filed February 4, 2025.

FIELD

This disclosure relates to medical imaging.

BACKGROUND

Intra-cardiac Echocardiography (ICE) is a key imaging modality for Electrophysiology (EP) procedures and Structural Heart Disease (SHD) interventions, providing real-time, high-resolution visualization of intracardiac structures. ICE catheter manipulation presents challenges due to limited direct visualization and frequent adjustments, making consistent and accurate device tracking difficult. However, in EP procedures, accurate catheter tip tracking is essential for precise lesion formation, while in SHD interventions, ICE aids in positioning structural therapy devices such as mitral clips, occluders, and valve implants.

SUMMARY

By way of introduction, the preferred embodiments described below include methods, systems, instructions, and/or computer readable media for an AI-based guidance system for intra-cardiac echocardiography manipulation to maintain continuous therapy device tip visibility.

In a first aspect, a system for tracking a therapy device tip during an Intra-cardiac Echocardiography (ICE) procedure, the system comprising: an ICE catheter configured to acquire an ICE imaging sequence of a patient during the ICE procedure; and a control unit comprising at least a processor and a memory, the control unit configured to track the therapy device tip in real time within the ICE imaging sequence using a tip tracking model, the tip tracking model comprising: a pre-trained ultrasound foundation model configured for feature extraction from images of the ICE imaging sequence; and a transformer based model configured to estimate an incident angle and a passing point of the therapy device tip in the ICE imaging sequence.

In a second aspect, a method for configuring a transformer based model for tip tracking, the method comprising: providing a pre-trained ultrasound foundation model; acquiring a hybrid training dataset of a plurality of ICE imaging sequences comprising clinical sequences and synthetic sequence data; training the transformer based model for a plurality of iterations, wherein each iteration comprises: extracting features from an ICE imaging sequence of the ICE imaging sequences by the pre-trained ultrasound foundation model; inputting the features into the transformer based model configured with one or more encoders that model temporal dependencies via self-attention; outputting, by the transformer based model, a next-frame position and angle; comparing the next-frame position and angle to ground truth positions and angles using a using Mean Squared Error (MSE) loss; and updating attention weights and embeddings of the transformer based model to refine temporal consistency and spatial accuracy.

In a third aspect, a method for tracking a tip of a therapy device, the method comprising: acquiring, by a ICE catheter, an ICE imaging sequence during an ICE procedure; estimating, in real time by a control unit, a position and orientation of the tip of the therapy device during the ICE procedure, wherein the position and orientation are estimated by an ultrasound foundation model and transformer based deep learning model; and controlling the ICE catheter during the ICE procedure by a robotic catheter system based at least in part of the estimated position and orientation of the tip of the therapy device.

Any one or more of the aspects described above may be used alone or in combination. These and other aspects, features and advantages will become apparent from the following detailed description of preferred embodiments, which is to be read in connection with the accompanying drawings. The present invention is defined by the following claims, and nothing in this section should be taken as a limitation on those claims. Further aspects and advantages of the invention are discussed below in conjunction with the preferred embodiments and may be later claimed independently or in combination.

BRIEF DESCRIPTION OF THE DRAWINGS

The components and the figures are not necessarily to scale; emphasis instead being placed upon illustrating the principles of the embodiments. Moreover, in the figures, like reference numerals designate corresponding parts throughout the different views.

Figure 1 depicts an example system for tracking a therapy device tip during an Intra-cardiac Echocardiography (ICE) procedure according to an embodiment.

Figure 2 depicts an example architecture of a model for tracking a therapy device tip during an Intra-cardiac Echocardiography (ICE) procedure according to an embodiment.

Figure 3 depicts an example method for training a model for tracking a therapy device tip during an Intra-cardiac Echocardiography (ICE) procedure according to an embodiment.

Figure 4 depicts an example of an entry angle, rotation angle, and tip passing location.

Figure 5 depicts an example method for applying a model for tracking a therapy device tip during an Intra-cardiac Echocardiography (ICE) procedure according to an embodiment.

Figure 6 depicts an example of predicted angles and locations of a therapy device according to an embodiment.

Figure 7 depicts an example neural network according to an embodiment.

DETAILED DESCRIPTION

Embodiments described herein provide a model that estimates an incident angle and trajectory of therapy devices within ICE images in real time in order to provide guidance for ICE manipulation to maintain continuous therapy device tip visibility. An ICE imaging sequence is acquired. A pre-trained ultrasound foundation model is used to extract spatial and temporal features in the sequence of ICE images. A transformer based temporal deep learning model is used that integrates the extracted spatial and temporal features while incorporating historical passing points and angular information to in order to track the catheter tip within the ICE imaging plane. The transformer based temporal deep learning model improves upon existing incident angle and trajectory model estimations in ICE procedures by improving prediction accuracy and consistency.

The proposed transformer based temporal deep learning model differs from and improves upon existing solutions in several ways. Unlike traditional manual adjustments or hardware-dependent EM tracking, the described methods and systems predict the catheter tip position and incident angle in real time using an innovative AI model. Unlike previous deep learning approaches, this system leverages a transformer network for sequential ICE frame analysis, improving accuracy and stability. The model is trained on a combination of real clinical sequences and synthetic datasets, addressing anatomical variability and ensuring robustness. The model provides automated guidance for robotic ICE catheter adjustments, ensuring that the device tip remains visible throughout the procedure. These advantages enable greater procedural accuracy, reduced operator workload, and enhanced clinical outcomes in ICE-guided interventions.

Intra-cardiac Echocardiography (ICE) is a crucial imaging modality for Electrophysiology (EP) procedures and Structural Heart Disease (SHD) interventions. ICE is an imaging technique that uses an ultrasound catheter inserted into the heart to provide real-time, high-resolution images of cardiac structures. ICE may be performed with the patient under conscious sedation, without the need for endotracheal intubation. Unlike traditional transthoracic or transesophageal echocardiography, which relies on external or semi-invasive approaches, ICE employs a catheter-based probe inserted directly into the heart or nearby vasculature. This proximity to the heart provides high-resolution, real-time images of intracardiac anatomy, intracardiac physiology, and therapy devices used in various procedures. The ICE catheter may be a thin, flexible tube equipped with an ultrasound transducer at its tip, which can emit and receive sound waves to visualize intracardiac structures and therapy devices in real-time. Steering controls may allow a clinician or robotic system to precisely navigate and position the catheter in the heart.

ICE imaging may be used for multiple different procedures such as guiding catheters for mapping and ablation of arrhythmias, and/or guiding devices for septal defect closures (ASD/PFO closures), atrial appendage closure (LAAC), and transcatheter aortic valve replacement (TAVR) among other uses. ICE ablation is used as an example below, but any procedure where the tip location and orientation is used for control may make use of the model as described herein. ICE ablation is a technique in which ICE ultrasound imaging is used during cardiac ablation to visualize and guide the catheter placement, allowing for precise tissue contact, identification of anatomical structures, and early detection of complications like pericardial effusion. The real-time imaging helps identify abnormal heart tissue (substrate), improve the accuracy of creating scar tissue to block faulty signals, and reduces fluoroscopy time, procedure duration, and recurrence of arrhythmias in some cases. For ICE ablation, the left atrium may be the most common anatomy of interest for this context. Anatomic structures includes the left atrial appendage (LAA), left inferior pulmonary vein (LIPV), left superior pulmonary vein (LSPV), right inferior pulmonary vein (RIPV), and right superior pulmonary vein (RSPV). Additional, different, or fewer structures or parts of interest may be included. Any of the structures may be imaged, such as a root or base of a vein. The pulmonary veins, specifically their junction with the left atrium and the area around the pulmonary vein ostium or other heart chambers may be the anatomy of interest in other embodiments. Other imaging locations may result in other anatomy of interest, such as arteries or veins.

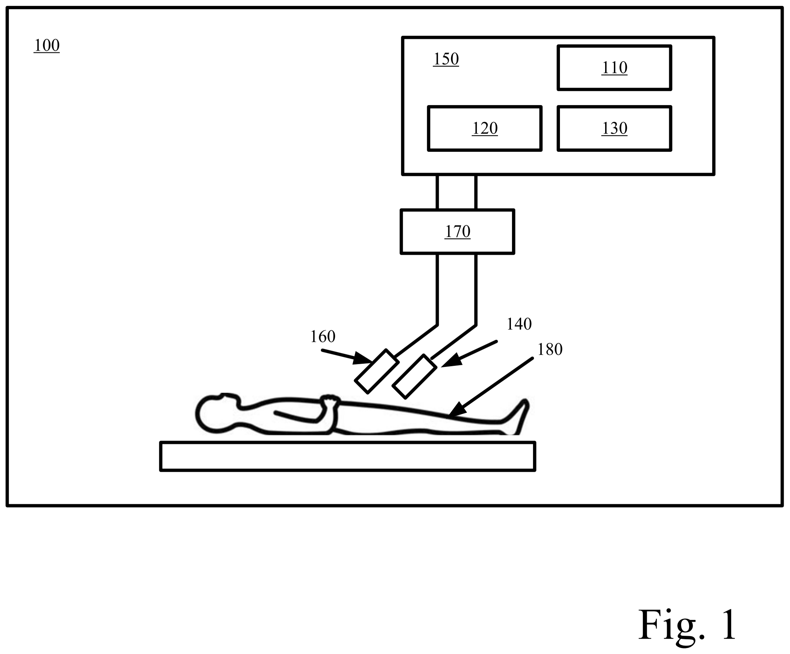

Figure 1 depicts an example ICE system 100 for use in electrophysiology procedures such as ICE ablation. The system 100 includes an ICE catheter 140, a therapy device 160, a control unit 150, and optionally a robotic catheter control system 170. The ICE catheter 140 and/or therapy devices 160 may be controlled using the robotic catheter control system 170. Alternatively, the ICE catheter 140 and/or therapy devices 160 may be controlled by a clinician during a procedure. The ICE catheter 140 is configured to acquire imaging data of a patient's tissue and/or organs. The imaging data may also include the therapy device 160. The therapy device 160 (for example, an ablation catheter) is configured to be inserted into the patient 180 in order to perform one or more therapeutic functions. The control unit 150 may process the imaging data in real time to estimate the location and orientation of the ICE catheter 140 or therapy device 160 in particular the incident angle and trajectory of the therapy device(s) 160 using an improved model as described herein that incorporates both an ultrasound foundation model and a temporal transformer based deep learning model.

In an example, the ICE system 100 includes an ICE catheter 140 that is configured to deliver high-resolution, real-time imaging of intracardiac and pericardiac structures. In an embodiment, the ICE catheter 140 integrates a phased array ultrasound transducer capable of producing 2D, 3D, and/or 4D volumetric imaging. The 4D (3D + time) capability allows for real-time visualization of dynamic cardiac structures, such as heart valves, septa, and blood flow patterns and real-time visualization of therapy devices 160, providing critical spatial and temporal information for tracking and/or monitoring a therapy device 160 . One of the key advantages of ICE is its ability to provide real-time guidance during minimally invasive cardiac procedures. For example, during catheter-based interventions, ICE facilitates precise catheter positioning and visualization of ablation targets. Similarly, ICE helps delineate anatomical landmarks and verify device placement. By providing direct intracardiac visualization, ICE eliminates the need for external imaging modalities like fluoroscopy in certain cases, thereby reducing radiation exposure to both the patient and the medical team.

The therapy device(s) 160 may include any medical device that is inserted into the patient such as mitral clips, occluders, and valve implants. Mitral clips approximate mitral valve leaflets to reduce regurgitation without open-heart surgery. Occluders close atrial septal defects, patent foramen ovale, or left atrial appendage openings to prevent shunting or embolic events. Valve implants replace or reinforce native or bioprosthetic valves. The therapy device 160 may also include structural heart therapy devices used in transcatheter procedures such as atrial septal defect (ASD) closure, patent foramen ovale closure, left atrial appendage (LAA) occlusion, and transcatheter valve repair or replacement. The therapy device 160 may also be an interventional vascular devices such as used in thrombectomy, endovascular stent deployment, or vena cava filter placement. The therapy device 160 may also be a drug or energy delivery catheters that provides local pharmacologic agents, gene therapy vectors, or ultrasound-mediated energy for tissue modulation. Alternative therapy devices 160 may be used.

In an embodiment, the ICE catheter 140 and/or therapy device 160 (such as an ablation catheter) is controlled using a robotic catheter navigation system 170. In an example, the robotic catheter navigation system 170 includes a catheter, a base, a catheter handle housing, an access point base, an access point guide, and an arm. In an embodiment, the robotic catheter navigation system 170 controls the ICE catheter 140 in order to acquire images of the therapy device 160. In another embodiment, the therapy device 160 is also controlled by the robotic catheter navigation system 170, for example in the case of an ablation catheter. Other catheters may be controlled by the robotic catheter navigation system 170. The base and catheter handle housing form a handle robot. The access point base and access point guide form an access point robot. The arm connects or links the handle robot to the access point robot. The cable interfaces with an ultrasound device for, e.g., image processing, beam forming, displaying the generated image, etc. The robotic catheter navigation system 170 is only one example. Other configurations of robotic catheter navigation system 170 are possible. In an embodiment, the robot controls the navigation and trajectory planning for the ICE catheter 140. As the ICE catheter 140 moves, the closed-loop nature of a robot control algorithm supervises the progress of the robot and continually monitors the system for errors and deviation from the target.

In an embodiment, as the ICE catheter 140 is moved within the patient 180, image data is acquired and processed to estimate an incident angle and trajectory of a therapy device 160 within the ICE images in real time so that the ICE catheter 140 is able to continuously visualize the therapy device 160 throughout a procedure. In previously used systems, electromagnetic (EM) sensor-based tracking may be used where systems incorporate EM sensors to track catheter and other device positions. These systems, however, require additional hardware and are susceptible to magnetic field interference. Robot-assisted ICE catheter 140 control has been explored to enhance accuracy and reduce operator workload. However, for effective robotic assistance, accurately estimating the direction and angle of devices as they appear in ICE images is critical in guiding the automated movement of the ICE catheter 140. Existing methods lack such robust real-time guidance for device tip tracking. There are several reasons why catheter tip tracking is a difficult problem including, for example, the requirement of maintaining continuous visibility of the therapy device tip within the ICE imaging field. Frequent adjustments and limited direct visualization make it difficult to consistently track device tips. For example, due to the dynamic nature of the procedure, in EP procedures, the tracking of catheter tip tracking is often lost. In SHD interventions devices such as mitral clips, occluders, and valve implants require careful alignment, which is complicated when the device moves out of the ICE imaging plane.

Embodiments described herein provide an innovative AI-driven model for estimating the incident angle and trajectory of therapy devices 160 within ICE images, ensuring continuous visualization and reducing operator workload. The innovative AI-driven model improves upon previous AI attempts by incorporating a pre-trained ultrasound foundation model to extract features and a transformer based model 220 that estimates the incident angle and trajectory of a therapy device 160.

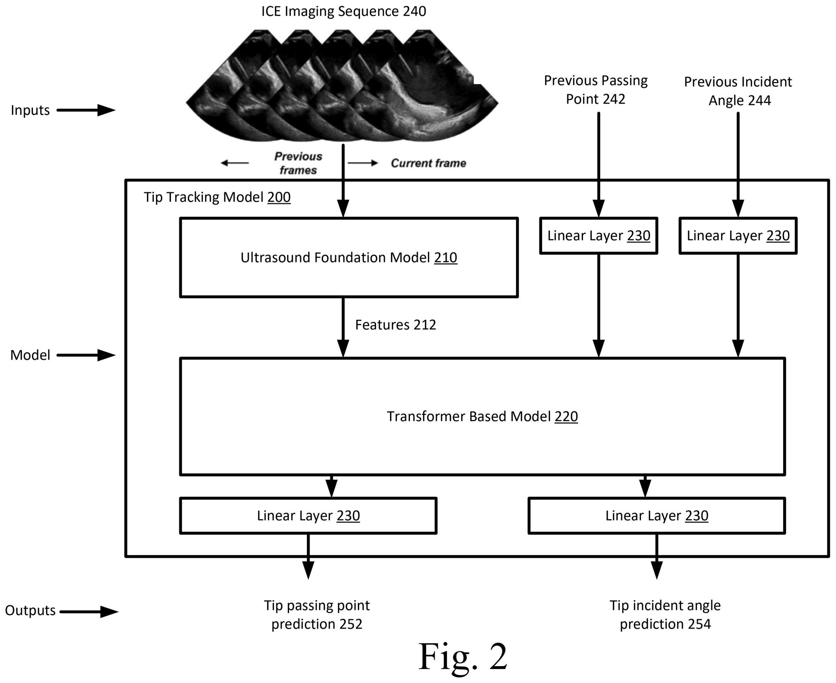

Figure 2 depicts an example of the AI-Driven Model Architecture and Processing Pipeline of the tip tracking model 200. As depicted in Figure 2, the AI driven model architecture of the tip tracking model 200 includes an ultrasound foundation model 210, a transformer based model 220, and a plurality of linear layers 230 for predicting a tip passing point 252 for predicting a tip incident angle prediction 254. The input is an ice imaging sequence 240 and a previous passing point 242 and incident angle 244 where available. The output is the tip passing point prediction 252 and the tip incident angle prediction 254. Alternative architectures may be used, for example where additional components or layers may be included. The image data from the ICE catheter 140 may be preprocessed to a defined resolution. Further processing may be performed on the input data and the outputs depending on the procedures and analytic requirements. For example, for a robotic catheter control system 170 additional data may be output or the output data may be further processed for automatic control of the catheter 140.

The ultrasound foundation model 210 is configured for feature 212 extraction from input ICE images, e.g. the ICE imaging sequence 240 acquired by the ICE catheter 140. In an embodiment, the ultrasound foundation model 210 is a large-scale neural network trained on vast and diverse datasets using self-supervised or unsupervised learning to develop broadly transferable representations. Instead of being designed for a single task, the ultrasound foundation model 210 is configured to learn general patterns such as visual patterns that can be efficiently adapted to different downstream applications through additional adapters. For estimating the incident angle and trajectory of a therapy device 160, the ultrasound foundation model 210 processes raw data into latent feature embeddings capturing structural information. The ultrasound foundation model 210 may be pre-trained on learning invariances without explicit labels, enabling the model to generalize across heterogeneous domains.

In an embodiment, the ultrasound foundation model 210 is a vision foundation model designed to provide a unified framework for echocardiographic image analysis across multiple clinical applications. The ultrasound foundation model 210 employs a transformer based encoder architecture pre-trained on a large and diverse in-domain dataset of millions of echocardiographic images using self-supervised learning via the DINOv2 framework. Through this large-scale pretraining, the encoder learns generalizable spatial representations of cardiac structures, motions, and imaging characteristics independent of transducer type, vendor, or patient population. The framework is configured to support both full fine-tuning and parameter-efficient adaptation, allowing performance optimization with minimal computational resources. The pre-trained encoder remains consistent across tasks, ensuring a common representational basis for varied clinical objectives. Once pre-trained, the model 210 is able to extract features 212 from an input ICE imaging sequence 240. In certain embodiments, alternative models may be used for feature extraction.

As depicted in Figure 2, the extracted features 212 from the ICE imaging sequence 240 are input into the temporal transformer based model 220 that estimates, in real time, the incident angle 254 and passing point 252 of a therapy device 160. The transformer based temporal model 220 is configured to input data with inherent temporal dependencies. The transformer based temporal model 220 uses self-attention mechanisms to model relationships between time steps rather than relying on fixed receptive fields or recurrent structures. Each temporal token, representing a frame, timestep, or feature embedding, attends to all others, allowing the transformer based temporal model 220 to capture both short-term dynamics and long-range dependencies in a unified representation. Positional or temporal encodings are added to preserve sequence order, enabling the network to infer temporal progression.

The tip tracking model 200 is configured to capture both spatial and temporal dependencies associated with a moving therapy device 160 tip / catheter tip over time. In this configuration, each frame of the sequential ICE image stream 240 is first processed by the ultrasound foundation model 210 to extract spatial features describing the local region around the tip, including a bounding box encoding a passing point and an associated incident angle that characterizes the directional orientation of motion.

The passing point refers to the instantaneous spatial position where a distal tip or active segment of the therapy device 160 intersects or crosses the imaging plane of the ICE transducer. The passing point represents the device's projection within the 2D ultrasound view, corresponding to where its trajectory passes through the plane being imaged.

Because ICE captures a thin slice of cardiac anatomy, the passing point identifies the moment and location where the 3D motion of the device enters, exits, or traverses that 2D slice. The incident angle refers to the angle between the trajectory of the device's distal shaft or tip and the normal vector of the imaging plane at the point where the device intersects it. This angle quantifies how obliquely or perpendicularly the device crosses the ultrasound plane. A small incident angle indicates that the device is passing almost parallel to the plane, often appearing elongated or blurred, while a large angle indicates near-perpendicular intersection, where the tip appears as a compact or circular echo. The incident angle may be critical for interpreting the device's true 3D orientation from the imaging data. The incident angle influences how the device's reflection and geometry appear within the ICE image and affects localization accuracy.

The per-frame feature vectors are assembled into a temporal sequence that serves as input to a transformer model that includes multiple encoder layers and multi-head self-attention modules. The transformer encoder models inter-frame relationships by allowing each feature vector to attend to every other feature in the imaging sequence 240. An attention mechanism enables the network to learn long-range temporal dependencies, such as velocity changes, trajectory curvature, or transient occlusions. Temporal position embeddings preserve the order of frames, while a linear transformation layer integrates historical tip coordinates and angular information into the same feature space, ensuring consistent temporal alignment and robust motion continuity.

The model 200 is trained to predict the tip's subsequent bounding box coordinates and incident angle for each frame or for a short forecast horizon. The prediction process aggregates contextual information across the entire sequence, enabling the model to infer motion patterns even under noisy or partially visible conditions. Optimization is performed using a Mean Squared Error (MSE) loss, minimizing the difference between predicted and ground truth positions and angles. This objective encourages smooth, temporally coherent trajectories and accurate spatial localization.

In an embodiment, the encoder layers in the transformer based temporal model 220 form a hierarchical sequence-processing structure that progressively refines temporal and spatial feature representations. Each encoder layer includes multi-head self-attention and feed-forward components, combined with normalization and residual connections to preserve information flow. The self-attention component provides for each frame's feature to interact with all others, capturing global temporal dependencies, while the feed-forward component projects and nonlinearly transforms the attended features to enhance abstraction. Stacking multiple encoder layers allows the model to learn progressively higher-level temporal correlations, from immediate motion continuity to complex trajectory dynamics, ensuring stable and context-aware tracking performance.

The attention heads in the transformer based temporal model 220 operate as independent subspaces within the self-attention mechanism. Each attention head learns to focus on different aspects of the temporal and spatial dependencies across the feature sequence. Each attention head computes a weighted relationship between all pairs of time steps, providing for the model to capture diverse motion patterns such as short-term displacements, long-term trajectory trends, and directional changes. By combining outputs from multiple attention heads, the model aggregates complementary temporal cues into a unified representation. The multi-head structure enhances interpretability and overall predictive stability in sequential tracking tasks.

The linear transformation layers in the transformer based temporal model 220 serve as an integration and alignment mechanism for temporal and spatial information. The linear transformation layer projects heterogeneous inputs such as historical tip coordinates, bounding box features, and incident angles into a common embedding space compatible with the transformer encoder. By applying a learned affine mapping, the linear transformation layers ensure that positional and angular data contribute coherently to temporal feature representations. The linear transformation layer effectively encodes motion continuity and spatial geometry. This provides the transformer with context about directionality and past movement. This further facilitates improved prediction accuracy across sequential tracking frames.

The output of the model is a tip passing point prediction 252 and tip incident angle prediction 254. Unlike traditional CNN-based approaches, the model leverages attention mechanisms to capture spatial and temporal dependencies. The system provides continuous probe tracking, reducing the need for manual ICE catheter 140 repositioning during procedures. In an embodiment, the model 200 is trained using real and synthetic data. The combination of real clinical ICE sequences and synthetic augmentation ensures generalizability across different patient anatomies.

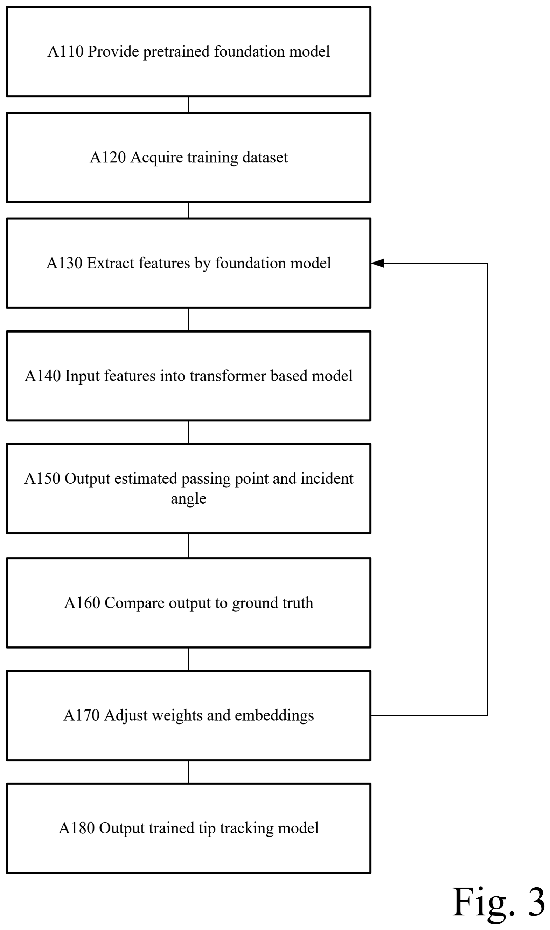

Figure 3 depicts an example workflow for training the tip tracking model 200. In an embodiment, the foundation model is pre-trained to extract features from an ultrasound sequence. The acts are performed by the system of Figures 1, 2, 7, other systems, a workstation, a computer, and/or a server. Additional, different, or fewer acts may be provided. The acts are performed in the order shown (e.g., top to bottom) or other orders. Certain acts may be omitted or changed depending on the results of the previous acts. The training of the transformer based model 220 involves sequentially optimizing the transformer based model 220 to predict accurate tip passing points 252 and incident angles 254 across time when provided extracted features 212 from the ultrasound foundation model 210. Input sequences containing spatial features, bounding box coordinates, and angular data are encoded and passed through multi-layer transformer encoders that model temporal dependencies via self-attention. A linear transformation layer integrates historical spatial and directional information before prediction. The model outputs the next-frame position and angle, which are compared to ground truth values using Mean Squared Error (MSE) loss. Gradients are backpropagated through the layers, updating attention weights and embeddings to refine temporal consistency and spatial accuracy across training iterations.

For training the model, at Act A110, a pre-trained ultrasound foundation model 210 is provided. The pre-trained ultrasound foundation model 210 is configured to extract features 212 from input ICE imaging data. In an embodiment, the ultrasound foundation model 210 functions as a domain-specific image encoder that converts each 2D ICE frame into a compact embedding suitable for temporal modeling.

At Act A120, a training dataset is acquired. In an embodiment, the training dataset includes both synthetic and real data. Acquiring a large-scale ICE dataset with accurate catheter tip locations and ground-truth incident angles may be challenging. To address this, the training dataset is acquired by using recorded clinical sequences and synthetic data generation to simulate various device orientations, incident angles, and anatomical interactions, ensuring clinical relevance. The recorded clinical sequences may be provided from actual scans of various patients across different demographics and operators. The synthetic data may be generated using various different processes, including physically simulated data and algorithmically simulated data.

In an embodiment, a water chamber is used to generate the synthetic sequences. Various device orientations and incident angles are simulated to reflect real-world anatomical interactions, ensuring that the training dataset covers a broad range of scenarios. To capture the catheter tip's position and orientation, both the ICE catheter 140 and device catheter tip are equipped with EM sensors, initializing the sensor frame such that ICE catheter 140 and tip heading aligns with the z-axis and ICE US fan direction aligns with the EM x-axis. The synthetic / simulated ICE images are collected in a water chamber against a black background, enabling automated annotation using computer vision methods. For the ground truth labels, the $a_{rot}$ may be inferred from the bounding box diagonal orientation, while the $a_{entry}$ is computed using EM sensor data by $E_{ice}^{tip} = (E_{world}^{ice})^{-1} E_{world}^{tip}$, where $E_{world}$ is the global frame, and $a_{entry}$ was derived from the z-axis angle in $E_{ice}^{tip}$. To enhance realism and prepare the data for the foundation model, the extracted tip images may be overlaid onto real ICE images from clinical ablation procedures, preserving motion continuity and introducing intensity variations for improved generalization. Each case includes sequential frames, ensuring continuous tip movement, essential for real-time tracking model training. To prevent overfitting, the synthetic tip sequences and real ICE images may be strictly separated between training and test datasets.

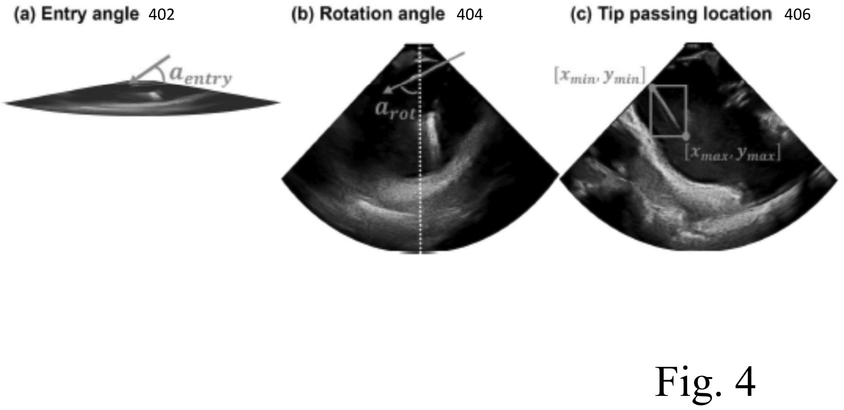

Figure 4 depicts an example of the dataset composition. In Figure 4, the entry angle $a_{entry}$ is shown in (a). This is the angle at which the tip enters the US fan area. In (b) the rotation angle $a_{rot}$ is depicted. $a_{rot}$ is the rotational angle between the tip and the center-line of the US image in 2D. In (c) the tip passing location is depicted which represents the position of the device within the 2D US image.

At Act A130, features are extracted from the hybrid training dataset by the pre-trained ultrasound foundation model 210. In an embodiment, a sequence of ICE images $I1:N = [i1, i2, ..., iN]$ is processed, resized, and passed through the Ultrasound foundation model 210 ($M_{foundation}$) to extract feature representations $FI = [f1, f2, ..., fN]$. To ensure temporal consistency, the prior passing point $B_{N-1}$ and the incident angle $A_{N-1}$ are projected into the same feature space via a linear transformation by a linear layer 230. In an embodiment, the ultrasound frames of an ICE imaging sequence 240 undergo echo-appropriate preprocessing, including cone masking, intensity normalization, and resizing. The pre-trained ultrasound foundation model 210 tokenizes each frame into patch tokens plus a classification (CLS) token and outputs feature embeddings that capture the anatomy and other features. A region of interest around the catheter tip or passing point may be cropped before encoding to emphasize task-relevant signal. Per frame, the CLS embedding or a pooled token representation is projected to a fixed dimensional vector. Bounding-box parameters and incident angle are linearly mapped into the same feature space and concatenated or fused with the visual embedding to form a frame descriptor. Consecutive frame descriptors form a sequence handed to the transformer temporal stack, which supplies positional encodings and self-attention across time.

At Act A140, the extracted features are input into the transformer based deep learning model 220 configured with one or more encoders that model temporal dependencies via self-attention. At Act A150, the transformer based deep learning model 220 outputs an estimated passing point and incident angle. In an embodiment, the passing point is represented as a bounding box as $B = [xmin, ymin, xmax, ymax]$, where $(xmin, ymin)$ and $(xmax, ymax)$ are the top-left and bottom-right coordinates. The incident angle is defined as $A = [a_{entry}, a_{rot}]$, where $a_{entry}$ is the entry angle into the 2D ICE image plane, and $a_{rot}$ denotes its rotational orientation. To ensure temporal consistency, a prior passing point $BN-1$ and prior incident angle $AN-1$ are projected into the same feature space via a linear transformation. The extracted features are concatenated with the CLS token and passed through a transformer network ($Mmain$) consisting of 8 encoder layers and 6 attention heads. The CLS output is processed via linear layers 230 to predict the passing point ^$B$ and incident angle ^$A$.

At Act A160, the estimated next-frame position and angle are compared to ground truth positions and angles using a using Mean Squared Error (MSE) loss. As described previously, the CLS output is processed via linear layers 230 to predict the passing point ^$B$ and incident angle ^$A$ where $B^, A^ = Mmain ([CLS, FI, BN_{-1}, AN_{-1}])$, $ltotal = lmse (B^, BN) +$

*lmse* (A^, AN). *BN* and *TN* are ground-truth values, and *lmse* is the MSE loss function. The MSE loss in the transformer based temporal model 220 quantifies the discrepancy between predicted and ground truth values of the tip's position and incident angle. The MSE loss computes the average of the squared differences across all predicted parameters, penalizing larger deviations more heavily to encourage precise and stable tracking. By minimizing MSE, the model learns to generate continuous and temporally consistent predictions that closely align with observed trajectories. The MSE loss function provides a smooth optimization landscape, promoting convergence toward accurate spatial localization and consistent angular estimation, which are critical for reliable motion tracking in dynamic or clinical environments.

At Act A170, attention weights and embeddings of the transformer based deep learning model 220 are updated to refine temporal consistency and spatial accuracy. The attention weights and embeddings may be updated using backprojection. In an embodiment, the weights of the pre-trained ultrasound model are frozen while the transformer based deep learning model 220 is configured. In other embodiment, the entire model may be trained end to end where the foundation model is pre-trained but updated / finetuned during the end to end training. The training process may be repeated multiple times to update the transformer based deep learning model 220 with each iteration changing the attention weights and embeddings until the transformer based deep learning model 220 is able to accurately estimate the next-frame position and angle. In an example, the model was trained for 117 epochs with a batch size of 6 in PyTorch on a single NVIDIA A100 GPU, achieving real-time performance at 25 [Hz]. Different GPUs and number of epochs (iterations) may be used. At Act A180, a trained model is output for use in an imaging procedure.

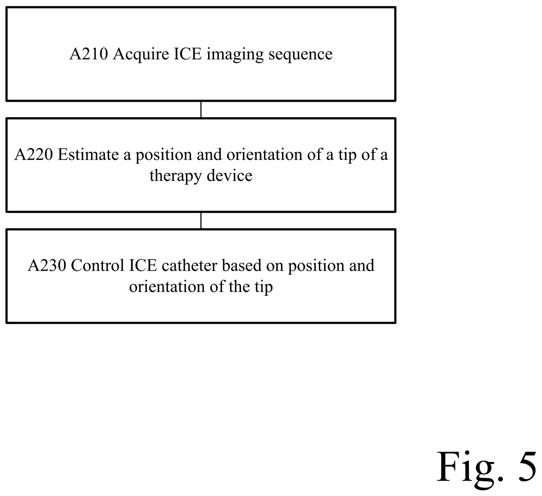

Figure 5 depicts an example method for tip tracking for a therapy device 160 during an ICE imaging procedure. The acts are performed by the system of Figures 1, 2, 7, other systems, a workstation, a computer, and/or a server. Additional, different, or fewer acts may be provided. The acts are performed in the order shown (e.g., top to bottom) or other orders. Certain acts may be omitted or changed depending on the results of the previous acts.

At Act A210, a ICE catheter 140 acquires an ICE imaging sequence 240 during an ICE procedure of a patient 180. The transducer of the ICE catheter 140 scans a plane. The scan plane is oriented based on a position of the catheter. As the catheter moves (e.g., translates or rotates), different scan planes are scanned. Each scan generates a frame of data representing the scan plane at that time. The frame of ultrasound data may be scalar values or display values (e.g., RGB) in a polar coordinate or Cartesian coordinate format. The frame of ultrasound data may be a B-mode, color flow, or other ultrasound image. A sequence of frames of data result from the ICE imaging representing an ICE sequence. Each frame represents a 2D scan plane, so a collection of frames representing different 2D scan planes in the volume of and/or around the heart are acquired. The ICE imaging sequence 240 may be displayed using a display while the ICE imaging sequence 240 is acquired. In an embodiment, the acquisition of the ICE imaging sequence 240 is performed by a robotic control system.

At Act A220 the control unit 150 estimates in real time while the ICE imaging sequence 240 is acquired, a position and orientation of an catheter tip of the ICE catheter 140 during the ICE procedure, wherein the position an orientation is estimated by a tip tracking model 200 applied by the control unit 150. The position and orientation may be derived from a tip passing point and tip incident angle provided by the model 200. The sequence of ICE images are processed by the tip tracking model 200 that includes a Ultrasound foundation model 210 and a temporal transformer based model 220. The input to the model is the sequence and prior passing points and incident angles. Features are extracted by the foundation model, combined and fed into the transformer based model 220 that predicts the final passing point and incident angle from distinct output layers.

In an embodiment, the Ultrasound foundation model 210 processes each frame of the ICE imaging sequence 240 to produce spatial feature embeddings. In addition, a temporal context stream ingests prior passing points and prior incident angles from recent timesteps. A learned linear projection maps these historical variables into the same embedding space used by the image features, encoding velocity, curvature, and directional continuity. Feature fusion occurs by concatenation or cross-attention between the spatial embeddings and the projected temporal embeddings, followed by addition of learned temporal position encodings. The sequence enters a transformer encoder stack, for example eight layers with six attention heads per layer. Causal masking enforces chronological flow, while multi-head attention exposes each timestep to global temporal context for robust handling of occlusions, speckle noise, and out-of-plane motion. Layer-norm and residual pathways stabilize optimization and sustain low-latency throughput. Two task-specific output heads operate on the final hidden states. A regression head predicts the passing point as bounding-box coordinates in image space or as calibrated 3D coordinates when paired with probe geometry. A second regression head predicts the incident angle around the tip's local tangent. Mean Squared Error loss over coordinates and angle supervises training.

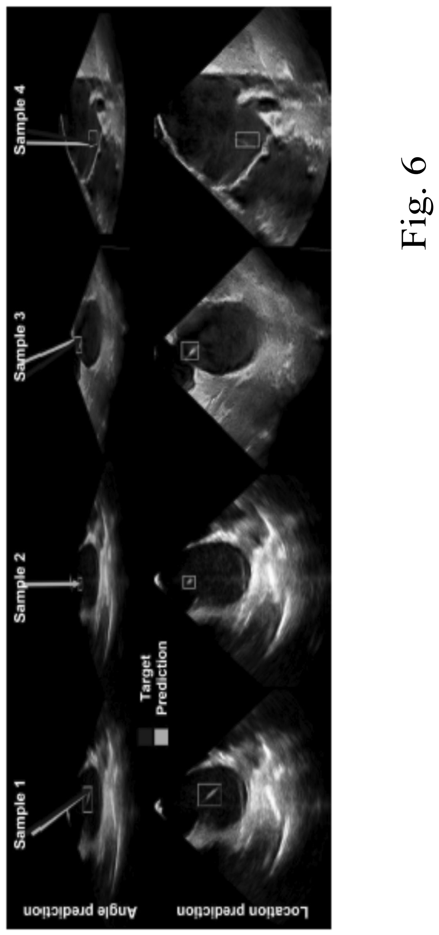

Figure 6 depicts an example output of the tip tracking model 200 including the estimate (prediction) of the angle and passing point (location). Figure 6 further dictates the target (ground truth) for each frame.

At Act A230 the ICE catheter 140 is controlled during the ICE procedure by the control unit 150, for example by directing a robotic catheter system 170 based at least in part of the estimated position and orientation of the catheter tip. The ICE catheter 140 may be controlled so that there is continuous visibility of the therapy device tip within the ICE imaging field during the procedure. In an embodiment, the control unit 150 inputs the updated position and orientation in real time (e.g., at video frame rate) and provides guidance to a robotic system or operator interface (UI), for example including confidence scores from attention-pooled uncertainty estimates provided by the model.

Referring back to Figure 1, the control unit 150 includes a processor 110, memory 120, and interface 130. The processor 110 may include an image processor that is configured to train, configured, and/or implement the models as described herein. The image processor 110 is a general processor, digital signal processor, three-dimensional data processor, graphics processing unit, application specific integrated circuit, field programmable gate array, artificial intelligence processor, digital circuit, analog circuit, combinations thereof, or another now known or later developed device for training and implementing models for tip tracking of a therapy device 160 in an ICE imaging sequence 240. The image processor 110 is a single device, a plurality of devices, or a network. For more than one device, parallel or sequential division of processing may be used. Different devices making up the image processor 110 may perform different functions. In one embodiment, the image processor 110 is also a control processor or other processor of an ICE imaging system. Other image processors of the ICE imaging system or external to the ICE imaging system may be used. The image processor 110 is configured by software, firmware, and/or hardware to process the data acquired by the imaging device and output one or more images.

The interface 130 includes an input device and an output device. The input may be an interface, such as interfacing with a computer network, memory 120, database, medical image storage, or other source of input data. The input may be a user input device, such as a mouse, trackpad, keyboard, roller ball, touch pad, touch screen, or another apparatus for receiving user input. The output is a display device but may be an interface. The display is a CRT, LCD, plasma, projector, printer, or other display device. The display is configured by loading an image to a display plane or buffer. The display is configured to display a reconstructed image(s) of the region of the patient 180. The interface may include a graphical user interface (GUI) enabling user interaction with the medical imaging device and enables user modification or selections in substantially real time.

The instructions for implementing the processes, methods, and/or techniques discussed herein are provided on non-transitory computer-readable storage media or memories, such as a cache, buffer, RAM, removable media, hard drive, or other computer readable storage media, for example the memory 120. The instructions are executable by the processor 110 or another processor 110. Computer readable storage media include various types of volatile and nonvolatile storage media. The functions, acts or tasks illustrated in the figures or described herein are executed in response to one or more sets of instructions stored in or on computer readable storage media. The functions, acts or tasks are independent of the instructions set, storage media, processor or processing strategy and may be performed by software, hardware, integrated circuits, firmware, micro code, and the like, operating alone or in combination. In one embodiment, the instructions are stored on a removable media device for reading by local or remote systems. In other embodiments, the instructions are stored in a remote location for transfer through a computer network. In yet other embodiments, the instructions are stored within a given computer, CPU, GPU, or system. Because some of the constituent system components and method steps depicted in the accompanying figures may be implemented in software, the actual connections between the system components (or the process steps) may differ depending upon the manner in which the present embodiments are programmed.

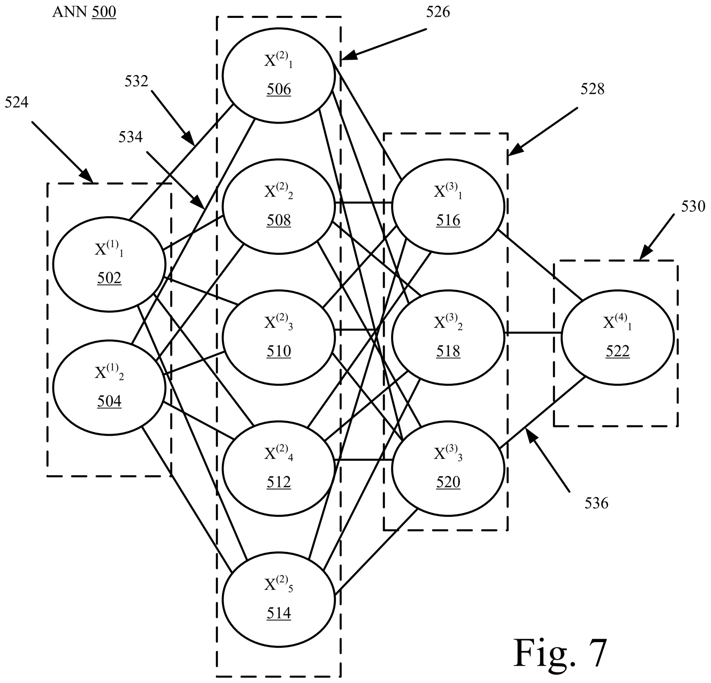

In an embodiment, the processor 110 implements one or more machine learning networks that are stored in the memory 120 in order to provide the tip tracking model 200. In particular, a machine learning network may comprise a neural network, for example a deep neural network, a convolutional neural network, or a convolutional deep neural network. The neural network may be an adversarial network, a deep adversarial network, a generative network, and/or a generative adversarial network. The network(s) are provided by or implemented with a neural network trained using deep learning. The network(s) may be defined as a plurality of sequential feature units or layers. Sequential is used to indicate the general flow of output feature values from one layer to input to a next layer. The information from the next layer is fed to a next layer, and so on until the final output. The layers may only feed forward or may be bidirectional, including some feedback to a previous layer. The nodes of each layer or unit may connect with all or only a sub-set of nodes of a previous and/or subsequent layer or unit. Skip connections may be used, such as a layer outputting to the sequentially next layer as well as other layers. Rather than pre-programming the features and trying to relate the features to attributes, the deep architecture is defined to learn the features at different levels of abstraction of the input data. The features are learned to reconstruct lower-level features (i.e., features at a more abstract or compressed level). Various units or layers may be used, such as linear, convolutional, pooling (e.g., max-pooling), deconvolutional, fully connected, or other types of layers. Within a unit or layer, any number of nodes is provided. For example, 100 nodes are provided. Later or subsequent units may have more, fewer, or the same number of nodes. In general, for convolution, subsequent units have more abstraction. Figure 7 shows an embodiment of an artificial neural network (ANN) 500, in accordance with one or more embodiments. Alternative terms for "artificial neural network" are "neural network", "artificial neural net" or "neural net". The artificial neural network 500 may be used in part in, for example, the one or more machine learning based networks utilized for the foundation model or the transformer based model 220 or a portion thereof. Alternatively, the foundation model and transformer based model 220 may use alternative architectures such as attention based mechanisms.

The artificial neural network 500 includes nodes 502-522 and edges 532, 534, ..., 536, wherein each edge 532, 534, ..., 536 is a directed connection from a first node 502-522 to a second node 502-522. In general, the first node 502-522 and the second node 502-522 are different nodes 502-522, it is also possible that the first node 502-522 and the second node 502-522 are identical. For example, in Figure 7, the edge 532 is a directed connection from the node 502 to the node 506, and the edge 534 is a directed connection from the node 504 to the node 506. An edge 532, 534, ..., 536 from a first node 502-522 to a second node 502-522 is also denoted as "ingoing edge" for the second node 502-522 and as "outgoing edge" for the first node 502-522.

In this embodiment, the nodes 502-522 of the artificial neural network 500 may be arranged in layers 524-530, wherein the layers may include an intrinsic order introduced by the edges 532, 534, ..., 536 between the nodes 502-522. In particular, edges 532, 534, ..., 536 may exist only between neighboring layers of nodes. In the embodiment shown in Figure 7, there is an input layer 524 including only nodes 502 and 504 without an incoming edge, an output layer 530 including only node 522 without outgoing edges, and hidden layers 526, 528 in-between the input layer 524 and the output layer 530. In general, the number of hidden layers 526, 528 may be chosen arbitrarily. The number of nodes 502 and 504 within the input layer 524 usually relates to the number of input values of the neural network 500, and the number of nodes 522 within the output layer 530 usually relates to the number of output values of the neural network 500.

In particular, a (real / complex) number may be assigned as a value to every node 502-522 of the neural network 500. Here, $x^{(n)}_i$ denotes the value of the i-th node 502-522 of the n-th layer 524-530. The values of the nodes 502-522 of the input layer 524 are equivalent to the input values of the neural network 500, the value of the node 522 of the output layer 530 is equivalent to the output value of the neural network 500. Furthermore, each edge 532, 534, ..., 536 may include a weight being a real number, in particular, the weight is a real number within the interval [-1, 1] or within the interval [0, 1]. Here, $w^{(m,n)}_{i,j}$ denotes the weight of the edge between the i-th node 502-522 of the m-th layer 524-530 and the j-th node 502-522 of the n-th layer 524-530. Furthermore, the abbreviation $w^{(n)}_{i,j}$ is defined for the weight $w^{(n,n+1)}_{i,j}$.

In particular, to calculate the output values of the neural network 500, the input values are propagated through the neural network. In particular, the values of the nodes 502-522 of the (n+1)-th layer 524-530 may be calculated based on the values of the nodes 502-522 of the n-th layer 524-530 by

$$x^{(n+1)}_j = f\left(\sum_i x^{(n)}_i \cdot w^{(n)}_{i,j}\right).$$

Herein, the function f is a transfer function (another term is "activation function"). Known transfer functions are step functions, sigmoid function (e.g. the logistic function, the generalized logistic function, the hyperbolic tangent, the Arctangent function, the error function, the smoothstep function) or rectifier functions. The transfer function is mainly used for normalization purposes.

In particular, the values are propagated layer-wise through the neural network, wherein values of the input layer 524 are given by the input of the neural network 500, wherein values of the first hidden layer 526 may be calculated based on the values of the input layer 524 of the neural network, wherein values of the second hidden layer 528 may be calculated based in the values of the first hidden layer 526, etc.

In order to set the values $w^{(m,n)}_{i,j}$ for the edges, the neural network 500 has to be trained using training data. In particular, training data includes training input data and training output data (denoted as $t_i$). For a training step, the neural network 500 is applied to the training input data to generate calculated output data. In particular, the training data and the calculated output data include a number of values, said number being equal with the number of nodes of the output layer.

In particular, a comparison between the calculated output data and the training output data is used to recursively adapt the weights within the neural network 500 (backpropagation algorithm). In particular, the weights are changed according to

$$w'^{(n)}_{i,j} = w^{(n)}_{i,j} - \gamma \cdot \delta^{(n)}_j \cdot x^{(n)}_i$$

wherein y is a learning rate, and the numbers $\delta^{(n)}_j$ may be recursively calculated as

$$\delta^{(n)}_j = \left(\sum_k \delta^{(n+1)}_k \cdot w^{(n+1)}_{j,k}\right) \cdot f'\left(\sum_i x^{(n)}_i \cdot w^{(n)}_{i,j}\right)$$

based on $\delta^{(n+1)}_j$, if the (n+1)-th layer is not the output layer, and

$$\delta_j^{(n)} = \left( x_k^{(n+1)} \cdot t_j^{(n+1)} \right) \cdot f' \left( \sum_i x_i^{(n)} \cdot w_{i,j}^{(n)} \right)$$

if the (n+1)-th layer is the output layer 530, wherein f' is the first derivative of the activation function, and $t^{(n+1)}_j$ is the comparison training value for the j-th node of the output layer 530.

In an embodiment, the transformer model's attention layers assess and use the specific context of each part of the imaging data sequence in multiple ways. The model reads the input image data sequences and converts them into vector embeddings, in which each element in the sequence is represented by its own feature vector(s) that numerically reflect features. The model then determines similarities, correlations and other dependencies (or lack thereof) between each vector and each other vector. The relative importance of one vector to another may be determined by computing the dot product between each vector. If the vectors are well aligned, multiplying them together will yield a large value. If the vectors are not aligned, their dot product will be small or negative. The alignment scores may then be converted into attention weights. This is achieved by using alignment scores as inputs to a softmax activation function, which normalizes all values to a range between 0-1 such that they all add up to a total of 1. For example, assigning an attention weight of 0 between "Vector A" and "Vector B" means that Vector B should be ignored when making predictions about Vector A. Assigning Vector B an attention weight of 1 means that it should receive 100% of the model's attention when making decisions about Vector A. The attention weights are used to emphasize or de-emphasize the influence of specific input elements at specific times. The network used for transformer based temporal modeling for tip tracking is derived from a Vision Transformer (ViT) network. Both architectures share the transformer backbone and self-attention mechanism, but they differ in structural intent and data representation. A ViT processes spatial tokens extracted from a single image, learning relationships among image patches to capture global spatial context. In contrast, the temporal transformer network as described herein extends this concept to sequential data, where each token represents a feature vector from a frame or time step rather than a spatial patch.

While the present invention has been described above by reference to various embodiments, it may be understood that many changes and modifications may be made to the described embodiments. It is therefore intended that the foregoing description be regarded as illustrative rather than limiting, and that it be understood that all equivalents and/or combinations of embodiments are intended to be included in this description. Independent of the grammatical term usage, individuals with male, female or other gender identities are included within the term.

The following is a list of non-limiting illustrative embodiments disclosed herein: Illustrative embodiment 1: A system for tracking a therapy device tip during an Intra-cardiac Echocardiography (ICE) procedure, the system comprising: an ICE catheter configured to acquire an ICE imaging sequence of a patient during the ICE procedure; and a control unit comprising at least a processor and a memory, the control unit configured to track the therapy device tip in real time within the ICE imaging sequence using a tip tracking model, the tip tracking model comprising: a pre-trained ultrasound foundation model configured for feature extraction from images of the ICE imaging sequence; and a transformer based model configured to estimate an incident angle and a passing point of the therapy device tip in the ICE imaging sequence.

Illustrative embodiment 2. The system of a previous illustrative embodiment, further comprising: a robotic control system for controlling the ICE catheter based at least in part on the estimated incident angle and passing point of the therapy device tip.

Illustrative embodiment 3. The system of illustrative embodiment 2, wherein the robotic control system is configured to control the ICE catheter to maintain continuous visibility of the therapy device tip within an ICE imaging field.

Illustrative embodiment 4. The system of a previous illustrative embodiment, further comprising: a display configured to display the ICE imaging sequence.

Illustrative embodiment 5. The system of a previous illustrative embodiment, wherein the pre-trained ultrasound foundation model is trained on millions of echocardiographic images using a self-supervised learning method.

Illustrative embodiment 6. The system of a previous illustrative embodiment, wherein the transformer based model comprises a plurality of encoder layers, a plurality of attention heads, and a plurality of linear layers.

Illustrative embodiment 7. The system of illustrative embodiment 6, wherein the transformer based model is trained using a MSE loss function where extracted features provided by the pre-trained ultrasound foundation are concatenated with a CLS token and passed through the transformer based model consisting of the plurality of encoder layers and the plurality of attention heads, wherein an output is processed by the plurality of linear layers to predict the passing point and the incident angle.

Illustrative embodiment 8. The system of illustrative embodiment 7, wherein a prior passing point and a prior incident angle are projected into a feature space via a linear transformation and input into the transformer based model.

Illustrative embodiment 9. The system of a previous illustrative embodiment, wherein the transformer based model is trained using a hybrid dataset set including clinical sequences and synthetic data generation.

Illustrative embodiment 10. The system of illustrative embodiment 9, wherein the synthetic data generation comprises data simulated using a water chamber.

Illustrative embodiment 11. A method for configuring a transformer based model for tip tracking of a therapy device, the method comprising: providing a pre-trained ultrasound foundation model; acquiring a hybrid training dataset of a plurality of Intra-cardiac Echocardiography (ICE) imaging sequences comprising clinical sequences and synthetic sequence data; training the transformer based model for a plurality of iterations, wherein each iteration comprises: extracting features from an ICE imaging sequence of the ICE imaging sequences by the pre-trained ultrasound foundation model; inputting the features into the transformer based model configured with one or more encoders that model temporal dependencies via self-attention; outputting, by the transformer based model, a next-frame position and angle; comparing the next-frame position and angle to ground truth positions and angles using a using Mean Squared Error (MSE) loss; and updating attention weights and embeddings of the transformer based model to refine temporal consistency and spatial accuracy.

Illustrative embodiment 12. The method of a previous illustrative embodiment, wherein the synthetic data comprises data simulated using a water chamber.

Illustrative embodiment 13. The method of a previous illustrative embodiment, wherein weights of the pre-trained ultrasound foundation model are frozen during the training of the transformer based model.

Illustrative embodiment 14. The method of a previous illustrative embodiment, wherein the transformer based model comprises a plurality of encoder layers, a plurality of attention heads, and a plurality of linear layers.

Illustrative embodiment 15. The method of illustrative embodiment 14, wherein the features provided by the pre-trained ultrasound foundation model are concatenated with a CLS token and input into the transformer based model consisting of the plurality of encoder layers and the plurality of attention heads, wherein an output is processed by the plurality of linear layers to predict the next-frame position and angle.

Illustrative embodiment 16. The method of illustrative embodiment 15, wherein a prior passing point and a prior incident angle are projected into a feature space via a linear transformation and input into the transformer based model with the concatenated features.

Illustrative embodiment 17. The method of a previous illustrative embodiment, wherein the plurality of iterations of training comprise one hundred or more iterations.

Illustrative embodiment 18. A method for tracking a tip of a therapy device, the method comprising: acquiring, by an Intra-cardiac Echocardiography (ICE) catheter, an ICE imaging sequence during an ICE procedure; estimating, in real time by a control unit, a position and orientation of the tip of the therapy device during the ICE procedure, wherein the position and orientation are estimated by an ultrasound foundation model and transformer based deep learning model; and controlling the ICE catheter during the ICE procedure by a robotic catheter system based at least in part of the estimated position and orientation of the tip of the therapy device.

Illustrative embodiment 19. The method of illustrative embodiment 18, wherein the ICE catheter is controlled so that the tip of the therapy device continuously is visualized in the ICE imaging sequence during the ICE procedure.

Illustrative embodiment 20. The method of illustrative embodiment 18, wherein the transformer based deep learning model comprises a plurality of encoder layers, a plurality of attention heads, and a plurality of linear layers.

**Claims**

1. A system for tracking a tip of a therapy device during an Intra-cardiac Echocardiography, ICE, procedure, the system comprising:

   an ICE catheter configured to acquire an ICE imaging sequence of a patient during the ICE procedure; and
   a control unit comprising at least a processor and a memory, the control unit configured to track the tip of the therapy device in real time within the ICE imaging sequence using a tip tracking model, the tip tracking model comprising:

      a pre-trained ultrasound foundation model configured for feature extraction from images of the ICE imaging sequence; and
      a transformer based model configured to estimate an incident angle and a passing point of the tip of the therapy device in the ICE imaging sequence.

2. The system of claim 1, further comprising:
   a robotic control system for controlling the ICE catheter based at least in part on the estimated incident angle and passing point of the tip of the therapy device.

3. The system of claim 2, wherein the robotic control system is configured to control the ICE catheter to maintain continuous visibility of the tip of the therapy device within an ICE imaging field.

4. The system of any one of claims 1 - 3, further comprising:
a display configured to display the ICE imaging sequence.

5. A method for configuring a transformer based model for tip tracking of a therapy device, the method comprising:

providing a pre-trained ultrasound foundation model;
acquiring a hybrid training dataset of a plurality of Intra-cardiac Echocardiography (ICE) imaging sequences comprising clinical sequences and synthetic sequence data;
training the transformer based model for a plurality of iterations, wherein each iteration comprises:

extracting features from an ICE imaging sequence of the ICE imaging sequences by the pre-trained ultrasound foundation model;
inputting the features into the transformer based model configured with one or more encoders that model temporal dependencies via self-attention;
outputting, by the transformer based model, a next-frame position and angle of a tip of the therapy device;
comparing the next-frame position and angle to ground truth positions and angles using a using Mean Squared Error (MSE) loss; and
updating attention weights and embeddings of the transformer based model to refine temporal consistency and spatial accuracy.

6. The method of claim 5, wherein the plurality of iterations of training comprise one hundred or more iterations.

7. A method for tracking a tip of a therapy device, the method comprising:

acquiring, by an Intra-cardiac Echocardiography (ICE) catheter, an ICE imaging sequence during an ICE procedure;
estimating, in real time by a control unit, a position and orientation of the tip of the therapy device during the ICE procedure, wherein the position and orientation are estimated by an ultrasound foundation model and transformer based deep learning model; and
controlling the ICE catheter during the ICE procedure by a robotic catheter system based at least in part of the estimated position and orientation of the tip of the therapy device.

8. The system or method of any one of claims 1 - 7, wherein the ICE catheter is controlled so that the tip of the therapy device continuously is visualized in the ICE imaging sequence during the ICE procedure.

9. The system or method of any one of claims 1 - 8, wherein the pre-trained ultrasound foundation model is trained on millions of echocardiographic images using a self-supervised learning method.

10. The system or method of any one of claims 1 - 9, wherein weights of the pre-trained ultrasound foundation model are frozen during the training of the transformer based model.

11. The system or method of any one of claims 1 - 10, wherein the transformer based learning model or deep learning model comprises a plurality of encoder layers, a plurality of attention heads, and/or a plurality of linear layers.

12. The system or method of claim 11, wherein the features provided by the pre-trained ultrasound foundation model are concatenated with a CLS token and input into the transformer based model consisting of the plurality of encoder layers and the plurality of attention heads, wherein an output is processed by the plurality of linear layers to predict the next-frame position and angle.

13. The system of method of claim 12, wherein a prior passing point and a prior incident angle are projected into a feature space via a linear transformation and input into the transformer based model with the concatenated features.

14. The system or method of claim 11, wherein the transformer based model is trained using a MSE loss function where extracted features provided by the pre-trained ultrasound foundation are concatenated with a CLS token and passed through the transformer based model consisting of the plurality of encoder layers and the plurality of attention heads, wherein an output is processed by the plurality of linear layers to predict the passing point and the incident angle of the tip of the therapy device.

15. The system or method of claim 14, wherein a prior passing point and a prior incident angle of the tip of the therapy device are projected into a feature space via a linear transformation and input into the transformer based model.

16. The system or method of an one of claims 1 - 15, wherein the transformer based model is trained using a hybrid dataset set, in particular an ICE imaging sequence, including clinical sequences and synthetic data generation, wherein the synthetic data generation, in particular, comprises data simulated using a water chamber.

Fig. 1

Fig. 2

A110 Provide pretrained foundation model

A120 Acquire training dataset

A130 Extract features by foundation model

A140 Input features into transformer based model

A150 Output estimated passing point and incident angle

A160 Compare output to ground truth

A170 Adjust weights and embeddings

A180 Output trained tip tracking model

Fig. 3

(a) Entry angle 402

(b) Rotation angle 404

(c) Tip passing location 406

$a_{entry}$

$a_{rot}$

$[x_{min}, y_{min}]$

$[x_{max}, y_{max}]$

Fig. 4

EP 4 785 875 A1

```
┌─────────────────────────────────────────────┐
│                                             │
│      A210 Acquire ICE imaging sequence      │
│                                             │
└─────────────────────────────────────────────┘
                      │
┌─────────────────────────────────────────────┐
│                                             │
│  A220 Estimate a position and orientation   │
│       of a tip of a therapy device          │
│                                             │
└─────────────────────────────────────────────┘
                      │
┌─────────────────────────────────────────────┐
│                                             │
│  A230 Control ICE catheter based on position│
│       and orientation of the tip            │
│                                             │
└─────────────────────────────────────────────┘
```

Fig. 5

Fig. 6

Fig. 7

ANN 500

$X^{(2)}_1$ 506

$X^{(2)}_2$ 508

$X^{(2)}_3$ 510

$X^{(2)}_4$ 512

$X^{(2)}_5$ 514

$X^{(1)}_1$ 502

$X^{(1)}_2$ 504

$X^{(3)}_1$ 516

$X^{(3)}_2$ 518

$X^{(3)}_3$ 520

$X^{(4)}_1$ 522

524

526

528

530

532

534

536

EP 4 785 875 A1

**EUROPEAN SEARCH REPORT**

Europäisches
Patentamt
European
Patent Office
Office européen
des brevets

Application Number

EP 26 15 5755

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | US 2020/390417 A1 (GIJSBERS GERARDUS HENRICUS MARIA [NL] ET AL) 17 December 2020 (2020-12-17) * paragraphs [0001], [0006], [0009], [0016] - [0018]; claims; figures * | 1-16 | INV. A61B8/08 A61B8/12 A61B8/00 A61B34/20 |
| A | US 2012/172724 A1 (HILL ANTHONY D [US] ET AL) 5 July 2012 (2012-07-05) * paragraphs [0008] - [0009], [0020] - [0023], [0027] - [0031]; claims; figures * | 1-16 | |
| A | US 2022/370034 A1 (CHEN ALVIN [US] ET AL) 24 November 2022 (2022-11-24) * paragraphs [0025] - [0026], [0029], [0037], [0046] - [0048], [0060]; claims; figures * | 1-16 | |
| A,P | JAEYOUNG HUH ET AL: "Guidance for Intra-cardiac Echocardiography Manipulation to Maintain Continuous Therapy Device Tip Visibility", ARXIV.ORG CORNELL UNIVERSITY LIBRARY, 201 OLIN LIBRARY CORNELL UNIVERSITY ITHACA, NY 14853, 8 May 2025 (2025-05-08), XP092016884, * Materials and Methods * | 1-16 | TECHNICAL FIELDS SEARCHED (IPC) A61B |

The present search report has been drawn up for all claims

| Place of search | Date of completion of the search | Examiner |
|---|---|---|
| Munich | 28 May 2026 | Mundakapadam, S |

## ANNEX TO THE EUROPEAN SEARCH REPORT
## ON EUROPEAN PATENT APPLICATION NO.

EP 26 15 5755

This annex lists the patent family members relating to the patent documents cited in the above-mentioned European search report.
The members are as contained in the European Patent Office EDP file on
The European Patent Office is in no way liable for these particulars which are merely given for the purpose of information.

28-05-2026

| Patent document cited in search report | | Publication date | Patent family member(s) | | Publication date |
|---|---|---|---|---|---|
| US 2020390417 | A1 | 17-12-2020 | CN | 111479510 A | 31-07-2020 |
| | | | EP | 3482690 A1 | 15-05-2019 |
| | | | EP | 3709889 A1 | 23-09-2020 |
| | | | JP | 7269929 B2 | 09-05-2023 |
| | | | JP | 2021502851 A | 04-02-2021 |
| | | | US | 2020390417 A1 | 17-12-2020 |
| | | | WO | 2019096810 A1 | 23-05-2019 |
| US 2012172724 | A1 | 05-07-2012 | CN | 103281965 A | 04-09-2013 |
| | | | EP | 2632340 A1 | 04-09-2013 |
| | | | JP | 5834094 B2 | 16-12-2015 |
| | | | JP | 6182194 B2 | 16-08-2017 |
| | | | JP | 2014501156 A | 20-01-2014 |
| | | | JP | 2016047262 A | 07-04-2016 |
| | | | US | 2012172724 A1 | 05-07-2012 |
| | | | WO | 2012091784 A1 | 05-07-2012 |
| US 2022370034 | A1 | 24-11-2022 | CN | 114430671 A | 03-05-2022 |
| | | | EP | 4033987 A1 | 03-08-2022 |
| | | | US | 2022370034 A1 | 24-11-2022 |
| | | | WO | 2021058288 A1 | 01-04-2021 |

EPO FORM P0459

For more details about this annex : see Official Journal of the European Patent Office, No. 12/82

## EP 4 785 875 A1

**Patent documents cited in the description**

- US 63753524